# EUROPEAN PATENT SPECIFICATION

(11) **EP 0 600 480 B1**
(45) Date of publication and mention of the grant of the patent: **06.09.2000**
(21) Application number: 93119439.3
(22) Date of filing: 02.12.1993
(51) Int. Cl.: C07K 14/755, C07K 1/36

(54) **Process for the extraction of factor VIII-von willebrand factor (FVIII:C-FVW) complex from total human plasma**
Verfahren zur Reinigung von Faktor VIII-von willebrand Faktor (FVIII:C-FVW) Komplex aus menschlichem Plasma
Procédé d'extraction de facteur VIII-von willebrand facteur (FVIII:C-FVW) complexe à partir de plasma humain

(30) Priority: 04.12.1992 IT MI922778
(43) Date of publication of application: 08.06.1994
(73) Proprietor: SCLAVO S.p.A., I-53100 Siena (IT); I.S.I. ISTITUTO SIEROVACCINOGENO ITALIANO S.P.A., Castelvecchio Pascoli (LUCCA) (IT)
(72) Inventor: Arrighi, Silvana, I-53010 Casciano di Murlo (Siena) (IT); Borri, Maria Giuseppina, I-53100 Siena (IT); Bucci, Enzo, I-02010 Cittaducale (Rieti) (IT)
(74) Representative: Gervasi, Gemma, Dr.

(56) References cited:
- EP-A- 0 303 329
- EP-A- 0 416 983
- EP-A- 0 468 181
- WO-A-86/02838
- WO-A-90/05140

## Description

### Technical field of the invention

The present invention relates to a process for the extraction of Factor VIII - von Willebrand Factor (FVIII:C - FvW) complex from total human plasma.

The process comprising unfreezing the plasma at room temperature after stabilization with an antiprotease and a suitable prediluition; then the selective absorption on anionic exchanger of the factors constituting the protrombinic complex (PTC), and the extraction, on anionic exchanger as well, of the (FVIII:C - FvW) complex.

The (FVIII:C - FvW) complex eluted from the column under suitable saline and pH conditions, after suitable stabilization, treatment with aluminium hydroxyde [Al(OH₃)] and viral inactivation is further purified by a cationic exchanger.

### State of the art

The necessity of having available larger amounts of FVIII concentrates, not only for the application of a suitable prophylactic treatment, but also in order that all haemophiliacs A in the world could be treated, made particularly interesting the setting up of processes which permit obtaining said product in increasing amounts. The methods known and used till now are essentially based on purification techniques starting from a cryoprecipitate, but the step for obtaining this intermediate fraction enriched in FVIII implies a loss of 40-50% of the protein (which partially remains in the plasma supernatant) and therefore the complete purification process for obtaining a product of suitable quality gives a yield usually lower than 250 IU of FVIII:C/litre of plasma.

In EP-A-303329 it is described a process for isolating coagulation factors, including FVIII and vWF for example from blood plasma and plasma products by means of liquid chromatography using particular adsorbent materials.

A process for obtaining Factor VIII from total plasma is described in EP-A-416 983.

However, such process requires an adjustment of the plasma pH which, even with addition of several stabilizers, can cause instability.

Moreover, said process requires a number of steps and the use of techniques which are certainly valid with regard to laboratory preliminary phases as those described above (see the use of 250 ml of plasma reported in the example), but which are absolutely unsuitable if applied on industrial scale.

### Detailed description of the invention

The present invention enables to overcome the above mentioned drawbacks by a process which allows the recovery of all plasmatic constituents, therefore obtaining a better use of plasma, and moreover allows obtaining the Factor VIII:C with a higher yield (in terms of IU/l of plasma) and higher purity (in terms of IU/mg of protein) of the methods until now described. Moreover, the thus obtained concentrate of Factor VIII:C - FvW is considerably safer in case of a possible contamination because it is possible to carry out viral inactivation of a previously purified solution.

A further advantage of the present process if compared with the state of the art resides in the possibility of sorting out Factor VII, even in large amounts (about 50%), thanks to the diluition carried out at the beginning and to the particular conditioning performed on the anionic exchange resin utilized. The process according to the present invention enables obtaining a FVIII:C - FvW with high purity (specific activity of 30 - 50 IU/mg of protein, and a yield of 400-600 IU/l of plasma), by adsorbing the FVIII directly from the total plasma, suitably diluted and stabilized, and not from the cryoprecipitate, by anionic exchange chromatography, and further by purifying the solution by cationic exchange chromatography.

The first condition for the purification process according to the invention is the stabilization of plasma, unfrozen at room temperature, with an antiprotease, e.g. lysine, or with other basic amino acids or amino acids containing tiolic or indolic groups, at the concentration of 5.5 - 10.5 g/l of plasma (preferably 7,3 g/l) and suitably diluted in sterile and apyrogenic distilled water, in a volume of about 1/2 - 1/10 (preferably 1/10) of the volume of the worked plasma. The plasma-antiprotease mixture is left stirring at a temperature of 18-22°C for about 10-30 min, then the factors constituting the Protombrinic Complex i.e. Factors II, VII, IX and X are extracted by adsorption in batch or through a chromatographic column with anionic exchange resin (for example DEAE-Sephadex A-50, DEAE Spherodex, DEAE Trisacryl, etc.).

The resin expanded and conditioned in a saline solution containing 0.1 - 0.15 M (preferably 0.13 M) sodium chloride pH 7.5 - 8.5 (preferably pH 8) is added to the stabilized and prediluted plasma in amounts of 1.0 - 1.5 g of anhydrous resin per litre of starting plasma (preferably 1.2 g/l), left under gentle stirring for about 30 min at temperature 18 - 22°C (preferably 20°C) and then removed by centrifugation or filtration.

PTC, and/or its single constituting factors, is eluted and further purified from the mixture "resin + PTC constituting Factors" for example as described in the European Patent Application 93104633.8 in the name of the same applicants. Moreover, it is also possible to collect Factor VII with considerable yield (until 50%) (see what reported in the example). The plasma supernatant (remaining after the PTC elimination) is furtherly stabilized with 0.3 - 1.0 IU/ml heparin (preferably 0.5 IU/ml) and then fed into a chromatographic column whose volume is about 1/10 - 1/30 (preferably 1/20) of the volume of plasma (said column being such to have a diameter/height ratio of 1 - 1/6), containing an anionic exchange resin (for example DEAE Sepharose FF, Fractogel DEAE o DMAE, DEAE Spherodex, etc.) conditioned in TC buffer consisting of: 10 - 30 mM (preferably 20 mM) Tris, 0.13 - 0.17 M (preferably 0.15 M) sodium chloride, 1 - 3 mM (preferably 2 mM) calcium chloride, pH 6.6 - 7.0 (preferably 6.8) at a flow rate of 15 - 20 cm/h (preferably 16.5 cm/h).

Not adsorbed or weakly bound proteins are removed by washing the resin with TC buffer in a volume of about 15 - 20 column beds (preferably 18) and/or till the absorbance monitored at weight lenght of 280 nm is not less than 0.200. The FVIII adsorbed on the resin is eluted with 5 - 7 column beds (preferably 6) of TE buffer consisting of: 10 - 30 mM (preferably 20 mM) Tris, 0,25 - 0.35 M (preferably 0.3 M) sodium chloride, 0.08 - 0.12 (preferably 0. 1M) glycine, 0.7 - 0.12 mM (preferably 0.86) lysine, 1 - 3 mM (preferably 2 mM) calcium chloride, pH 6.6 - 7.0 (preferably 6.8).

The solution mainly consists of a FVIII:C - FvW complex eluted in 1:1 ratio with a yield of about 85% and a purity of about 3.5 - 6.5 IU/mg protein.

A 2% water suspension of aluminium hydroxyde in a dose of 2 - 8 ml/l (preferably 5 ml/l) of eluate maintained to the same temperature of the eluate is added under low stirring to the eluate suitably stabilized with 0.3 - 1.0 IU/ml heparin (preferably 0.5 IU/ml) and 10 - 20 mg/ml (preferably 15 mg/ml) polyethylene glycole (PEG)4000 ; after being in contact for about 5 min under low stirring, the proteins adsorbed on aluminium hydroxyde are removed by centrifugation at low speed. The precipitate is removed while the supernatant containing the FVIII:C - FvW complex is filtered by clarifying filters. The obtained solution has a purity of about 8 IU/mg of protein and a recovery on the starting plasma of about 75%.

In order to restore the osmolarity and concentration conditions, the solution is concentrated at about 1/5 of the starting volume, diafiltered against 2 - 3 volumes of TDF1 buffer consisting of: 10 - 30 mM (preferably 20 mM) Tris, 0.11 - 0.15 M (preferably 0.13 M) sodium chloride, 0.08 - 0.12 (preferably 0.1M) glycine, 1 - 3 mM (preferably 2 mM) calcium chloride, pH 6.6 - 7.0 (preferably 6.8) and then further concentrated at about 1/2 of the volume (for this scope a hollow fiber system of 30000d cut off is used).

The solution can be frozen or subjected to a viral inactivation process. To this scope you can use: either a liquid phase pastorization for 10 hours at 60 ± 1°C in the presence of stabilizers (glycine 60 g/Kg of solution, lysine 145.9 g/Kg of solution, dihydrate calcium chloride 0.294 g/Kg of solution, sucrose 1.2 Kg/Kg of solution at pH 7.45 - 7.55) or a treatment with solvent/detergent (particularly by using a TNBP mixture at the concentration of 0.3% v/w and Tween 80 at the concentration of 1% v/w), keeping it under low stirring for 6 hours at 25°C. The solution of FVIII:C - FvW complex "virus free" after filtration on clarifying membranes, is fed into a chromatographic column whose volume is about 1/30 of the starting volume of plasma (which is such to have a diameter/height ratio of 1 - 1/2), containing a cationic exchage column (for example S-Sepharose FF, Fractogel TSK SP-650, Fractogel EMD-650, AG 50W, SP Trisacryl, etc.) or a resin derived with sulphonic groups (for example Matrex, etc.) or a resin functionalized with heparin (for example Heparin Sepharose), conditioned in TC buffer, at a flow rate of 15-25 cm/h.

Not adsorbed or weakly bound proteins are removed by washing the resin with about 6 column volumes of TC buffer. The FVIII:C - FvW complex adsorbed on the resin is eluted with about 4 column volumes of TE buffer, with a recovery step of about 90% and a purity of about 40 IU/ml. The FVIII solution at high purity is brought again to the physiological condition of the solution and correctly formulated by diafiltration versus 2.5 volumes of TDF2 diafiltration buffer consisting of: 0.11 - 0.15 M (preferably 0.13 M) sodium chloride, 0.08 - 0.12 (preferably 0.1 M) glycine, pH 6.8 - 7.2 (preferably 7.1), concentrated until the wished values of IU/ml, sterilely filtered, lyophilized and dispensed into vials according to the processes known in the technique. Further advantages and features of the process according to the invention will be more properly understood in the light of the following example.

### EXAMPLE

### STAGE 1

About 1000 l of frozen fresh human plasma are unfrozen with continuos stirring up to a temperature of 20°C in a vessel equipped with water cooling jacket at 30-35°C, and during the unfreezing 730 g of lysine solubilized in about 100 l of steril and apyrogenic distilled water are added. The plasma-antiprotease mixture is left stirring at a temperature of 20°C for about 15 min, then 1.2 Kg of DEAE-Sephadex A 50, previously swollen in saline solution at pH 8 ± 0.1, is added. The suspension is left under gentle stirring for 30 min at room temperature. When this phase has ended, the "resin + PTC Factors" mixture is removed by centrifugation and further processed in order to obtain PTC or its single constituents; in particular with regard to FII, FIX and FX factors see the European Patent Application 93104633.8 whilst the Factor VII purification is described as follow.

The plasma obtained after PTC remotion is further processed in order to obtain FVIII:C - FvW at high purity as described hereinafter.

### Factor VII purification

The "resin + PTC" mixture recovered from the centrifuges, as above said, after washing in 0.1 M saline solution of sodium chloride pH 8 ± 0.1, is further suspended in TL1 phosphate buffer consisting of: 0.01 - 0.03 M dibasic sodium phosphate, 0.01 - 0.03 M monobasic potassium phosphate, 0.14 - 0.1 M sodium chloride, pH 6.9 ± 0.1, in a volume of about 1/50 of the starting volume of plasma (i.e. between 15 and 30 litres). The suspension is left under stirring for about 30 min at 4°C, then the resin is recovered by centrifugation and processed for obtaining PTC or its single constituents as above described.

The supernatant solution containing about 15 - 25 IU of FVII/ml suitably stabilized with 0.5 IU/ml heparin and 0.5 mM lysine is subjected to a viral inactivation process by the solvent/detergent method (particularly using a TNBP mixture at the concentration of 0.3% v/w and Tween 80 at the concentration of 1% v/w, keeping it under gentle stirring for 6 hours at 25°C).

The FVII "virus free" solution after filtration on clarifying membranes is diluted in 1:1 ratio in TL2 buffer consisting of: 0.01 - 0.03 M dibasic sodium phosphate, 0.01 - 0.03 M monobasic potassium phosphate, pH 8 ± 0.1 and fed into a chromatographic column containing an anionic exchanger (DEAE Sepharose FF, etc.) whose volume is about 1/200 of the starting plasma volume (ca. 5 l) (the column is such to have a diameter/height ratio of 1 to 1/2), conditioned under TL2 buffer containing 0.06-0.1M sodium chloride, at a flow rate of about 15 cm/h. Non adsorbed or weakly bound proteins and surfactants employed for viral inactivation are removed by washing the resin first with TL2 buffer in a volume corresponding to ca. 10 column beds and then with a TL2 buffer containing sodium chloride in a volume corresponding to ca. 5 column beds. FVII adsorbed upon the resin is then eluted with 4 column beds of TL1 buffer with a recovery of about 80% (namely 11-16 IU of FVII/ml of solution) and with a purity of 8-15 UI FVII/mg of protein. The final solution suitably established with 0.4 IU/ml heparin after diafiltration against 1-2 volumes of buffer containing 5-15 mM sodium citrate and sodium chloride 0.12-0.14M, pH 7±0.1 is concentrated about four times or anyway up to the wished values of UI/ml, sterilely filtered, aseptically dispensed into vials and lyophilized.

### Purification of Factor VIII:C - FvW complex

Supernatant PTC plasma, obtained as above described, is further stabilized with heparin 0.5 UI/ml and then fed into a chromatographic column containing about 50 l of DEAE Sepharose FF, conditioned in TC buffer, at a flow rate of 16.5 cm/h. Non adsorbed or weakly bound proteins are removed by washing the resin with a TC buffer in a volume corresponding to ca. 18 column beds. The FVIII:C - FvW complex adsorbed upon the resin is eluted with 6 column beds of TE buffer and collected in the presence of the following stabilizers: 0.5 UI/ml heparin and 15 mg/ml PEG 4000.

### STAGE 2

150 ml of a 2% water suspension of aluminum hydroxyde, kept at the same temperature of the FVIII solution, are added under gentle stirring to the solution eluted and stabilized as described in the previous stage. After ca. 5 minutes of gentle stirring, the polluting proteins adsorbed by alumium hydroxyde are removed by centifugation (centrifuge type Westfalia BKA 2), the supernatant is filtered on clarifying filters and then concentrated to ca. 1/5 of the starting volume on an ultrafiltration system with hollow fibre cartridges with cut off 30000d, diafiltered against 2.5 volumes of TDF1 buffer and then further concentrated to ca. 1/2 of the volume.

Now the solution can be frozen or can undergo a process of viral inactivation.

### STAGE 3

Viral inactivation processes employ the conventional operating procedures previously described.

### STAGE 4

After filtration on clarifying membranes, the solution of "virus free" FVIII:C - FvW complex is fed into a chromatographic column containing ca. 30 l S-Sepharose FF previously equilibrated in TC buffer, at a flow rate of ca. 18 cm/h. Non adsorbed or weakly bound proteins are removed by washing the resin with ca. 6 column volumes of TC buffer. FVIII:C - FvW complex adsorbed upon the resin is eluted with ca. 4 column volumes of TE buffer. The solution is then brought again to its physiologic condition through diafiltration against 2.5 volumes of TDF2 buffer. The final solution is further concentrated up to the wished values of UI/ml, filtered on clarifying filters and sterilized on 0.2 um porosity filters.

The sterile solution is then aseptically dispensed into vials and frozen at -40°C.

The vials are then lyophilized by slowly raising temperatures from -40°C to +30°C at low pressure. In order to better guarantee safety with regard to possible viral contamination, the lyophilized product can undergo a thermal treatment for 30 min at 100°C in immersion.

Said step does not imply biochemical alterations in the solution, but a simple loss of 10% on the final yield.

## Claims

1. Process for the extraction of Factor VIII:C - FvW complex from total human plasma, wherein:
a) plasma, unfrozen at room temperature, is stabilized with an antiprotease or other basic amino acids or wino acids containing thiolic or indolic groups, diluted in sterile and apyrogenic distilled water, in a volume of about 1/2 - 1/10 of the volume of the plasma
b) the aforesaid mixture is treated with an anionic exchange resin suitably conditioned in order to separate the factors constituting the protrombinic complex (PTC)
c) PTC plasma supernatant is stabilized with heparin and then fed into a chromatographic column containing an anionic exchange resin suitably conditioned
d) Factor VIII:C - FvW von Willebrand complex adsorbed upon the column in the previous step is eluted and the thus collected solution is stabilized with heparin and polyethylene glycol and then treated with a Al(OH)₃ suspension
e) the supernatant containing Factor VIII:C - FvW is filtered and the osmolarity conditions are restored in the solution which then undergoes viral inactivation
f) the solution obtained as described in the previous point is fed into a chromatographic column containing a suitably conditioned cationic exchange resin
g) Factor VIII:C - FvW complex adsorbed upon the column in the previous step is eluted and the thus obtained solution is brought to its physiologic condition, concentrated, dispensed into vials and lyophilized.

2. A process according to claim 1 wherein in step (a) antiprotease is lysine in a concentration of 5.5-10.5 g/l diluted with sterile and apyrogenic distilled water in a volume corresponding to 1/2 - 1/10 of the volume of the plasma.

3. A process according to claim 1 wherein in step (c) PTC plasma supernatant is stabilized with heparin 0.3-1 UI/ml, the anionic exchange resin is conditioned in 10-30 mM Tris buffer, 0.13-0.17 M sodium chloride, 1-3 mM calcium chloride, pH 6.6-7.0 and the flow rate in the column is of 15-20 cm/h.

4. A process according to claim 1 wherein in step (d) Factor VIII:C - FvW complex is eluted with 10-30 mM Tris buffer, 0.25-0.35 M sodium chloride, 0.08-0.12 M glycine, 0.7-1.2 mM lysine, 1-3 mM calcium chloride, pH 6.6-7.0.

5. A process according to claim 1 wherein the cationic exchanger resin according to point (f) is conditioned with buffer: 10-30 mM Tris, 0.13-0.17 M sodium chloride, 1-3 mM calcium chloride, pH 6.6-7.0.

6. A process according to claim 1 wherein Factor VIII:C - FvW complex adsorbed upon cationic exchanger resin according to point (g) is eluted with buffer: 10-30 mM Tris, 0.25-0.35 M sodium chloride, 0.08-0.12 M glycine, 0.7-1.2 mM lysine, 1-3 mM calcium chloride, pH 6.6-7.0.

## Patentansprüche

1. Verfahren zur Extraktion von Faktor VIII:C - FvW-Komplex aus menschlichem Plasma, wobei
a) Plasma, ungefroren bei Raumtemperatur mit einer Antiprotease oder anderen basischen Aminosäuren oder Aminosäuren, die Thiol- oder Indol-Gruppen enthalten, die in sterilem oder apyrogenem destilliertem Wasser in einem Volumen von etwa 1/2 - 1/10 des Plasma-Volumens verdünnt sind, stabilisiert wird;
b) das oben genannte Gemisch mit einem Anionen-Austauscherharz, das in einer Kochsalzlosung, die 0,1 bis 0,15 M Natriumchlorid enthält, bei pH 7,5 bis 8,5 konditioniert worden ist, behandelt wird, um die Faktoren, die den Prothrombin-Komplex (PTC) bilden, abzutrennen;
c) der PTC-Plasma-Überstand mit Heparin stabilisiert wird und dann in eine Chromatographiesäule geführt wird, die ein Anionen-Austauscherharz enthält, das in einem Puffer bestehend aus 10 bis 30 mM Tris, 0,13 bis 0,17 M Natriumchlorid, 1 bis 3 mM Calciumchlorid, pH 6,6 bis 7,0, konditioniert worden ist;
d) Faktor VIII:C - FvW von Willebrand-Komplex, der in dem vorherigen Schritt an der Säule adsorbiert wurde, eluiert wird und die auf diese Weise gesammelte Lösung mit Heparin und Polyethylenglykol stabilisiert wird und dann mit einer Al(OH)₃-Suspension behandelt wird;
e) der Überstand, der Faktor VIII:C - FvW enthält, filtriert wird und die Osmolaritätsbedingungen in der Lösung wieder hergestellt werden, die dann eine Virus-Inaktivierung durchmacht;
f) die Lösung, die erhalten wird, wie es im vorangehenden Punkt beschrieben ist, durch eine Chromatographiesäule geführt wird, die ein in geeigneter Weise kontidioniertes Kationen-Austauscherharz enthält;
g) Faktor VIII:C - FvW-Komplex, der im vorherigen Schritt an der Säule adsorbiert wurde, eluiert wird und die so erhaltene Lösung in ihren physiologischen Zustand gebracht wird, konzentriert wird, in Phiolen verteilt und lyphilisiert wird.

2. Verfahren nach Anspruch 1, wobei in Schritt (a) die Antiprotease Lysin eine Konzentration von 5,5 bis 10,5 g/l, verdünnt mit sterilem und apyrogenem destilliertem Wasser in einem Volumen, das 1/2 bis 1/10 des Volumens des Plasmas entspricht, hat.

3. Verfahren nach Anspruch 1, wobei in Schritt (c) der PTC-Plasma-Überstand mit 0,3 bis 1 IE/ml Heparin stabilisiert wird, das Anionen-Austauscherharz in 10 bis 30 mM Tris-Puffer, 0,13 bis 0,17 M Natriumchlorid, 1 bis 3 mM Calciumchlorid, pH 6,6 bis 7,0 konditioniert ist und die Durchflußgeschwindigkeit der Säule 15 bis 20 cm/h ist.

4. Verfahren nach Anspruch 1, wobei in Schritt (d) der Faktor VIII:C - FvW-Komplex mit 10 bis 30 mM Tris-Puffer, 0,25 bis 0,35 M Natriumchlorid, 0,08 bis 0,12 M Glycin, 0,7 bis 1,2 mM Lysin, 1 bis 3 mM Calciumchlorid, pH 6,6 bis 7,0, eluiert wird.

5. Verfahren nach Anspruch 1, wobei das Kationen-Austauscherharz nach Punkt (f) mit Puffer: 10 bis 30 mM Tris, 0,13 bis 0,17 M Natriumchlorid, 1 bis 3 mM Calciumchlorid, pH 6,6 bis 7,0, konditioniert ist.

6. Verfahren nach Anspruch 1, wobei der Faktor VIII:C - FvW-Komplex, der gemäß Punkt (g) am Kationen-Austauscherharz adsorbiert ist, mit Puffer: 10 bis 30 mM Tris, 0,25 bis 0,35 M Natriumchlorid, 0,08 bis 0,12 M Glycin, 0,7 bis 1,2 mM Lysin, 1 bis 3 mM Calciumchlorid, pH 6,6 bis 7,0, eluiert wird.

## Revendications

1. Procédé pour l'extraction du Facteur VIII:C - complexe FvW du plasma humain total dans lequel:
a) le plasma dégelé à temperature ambiante, est stabilisé avec une anti-protéase ou d'autres amino-acides de base contenant des groupes thioliques ou indoliques dilués dans de l'eau distillée stérile apyrogène, dans un volume correspondant environ à 1/2 - 1/10 du volume du plasma
b) pour séparer les facteurs constituant le complexe de prothrombine (PTC) le mélange mentionné ci-dessus est traité avec une résine d'échange anionique dans une solution saline contenant 0,1 - 0,15 M de chlorure de sodium ayant un pH correspondant à 7,5 - 8,5
c) le plasma PTC surnageant est stabilisé avec de l'héparine et ensuite alimenté dans une colonne de chromatographie contenant une résine d'échange anionique conditionnée dans une solution tampon consistant en: 10-30 mM Tris, 0,13-0,17 M de chlorure de sodium, 1-3 mM chlorure de calcium ayant un pH correspondant à 6,6 -7,0
d) le Facteur VIII: c - complexe FvW von Willebrand absorbé sur la colonne pendant la phase précédente est élué et la solution obtenue est stabilisée avec de l'héparine, du polyéthylène glycol et ensuite traitée avec une suspension de Al (OH)₃
e) le surnageant contenant le Facteur VIII: c - FvW est filtré et les conditions d'osmolarité sont rétablies dans la solution qui est ensuite soumise à l'inactivation des virus
f) la solution obtenue comme décrit dans la phase précédente est mise dans une colonne de chromatographie contenant une résine d'échange cationique appropriée
g) le Facteur VIII:c - complexe FvW absorbé sur la colonne comme décrit dans la phase précédente est élué et la solution obtenue est portée à sa condition physiologique, concentrée, mise dans des ampoules et lyophilisée.

2. Un procédé selon la revendication 1 où dans la phase (a) l'anti-protéase est la lysine avec une concentration de 5,5-10,5 g/l diluée avec de l'eau distillée stérile apyrogène ayant un volume correspondant à 1/2-1/10 du volume du plasma.

3. Un procédé selon la revendication 1 où dans la phase (a) le plasma PTC surnageant est stabilisé avec 0,3-1 UI/ml d'héparine, la résine d'échange anionique est conditionnée dans une solution tampon consistant en 10-30 mM Tris, 0,13-0,17 M de chlorure de sodium, 1-3 mM de chlorure de calcium, ayant un pH correspondant à 6,6-7,0 et le débit dans la colonne correspondant à 15-20 cm/h.

4. Un procédé selon la revendication 1 où dans la phase (d) le Facteur VIII: c- complexe FvW est élué avec une solution tampon consistant en 10-30 mM Tris, 0,25-0,35 M de chlorure de sodium, 0,08-0,12 M de glycine, 0,7-1,2 mM de lysine, 1-3 mM de chlorure de calcium ayant un pH correspondant à 6,6-7,0.

5. Un procédé selon la revendication 1 où la résine d'échange cationique dans le point (f) est conditionnée avec une solution tampon composée par 10-30 mM Tris, 0,13-0,17 M de chlorure de sodium, 1-3 mM de chlorure de calcium, ayant un pH correspondant à 6,6-7,0.

6. Un procédé selon la revendication 1 où le Facteur VIII:c - complexe FvW est absorbé sur la résine d'échange cationique comme mentionné dans le point (g) est élué avec une solution tampon consistant en 10-30 mM Tris, 0,25-0,35 M de chlorure de sodium, 0,08-0,12 M de glycine, 0,7-1,2 mM de lysine, 1,3 mM de chlorure de calcium, ayant un pH correspondant à 6,6-7,0.
